# EUROPÄISCHE PATENTSCHRIFT

(11) **EP 1 567 076 B1**
(45) Veröffentlichungstag und Bekanntmachung des Hinweises auf die Patenterteilung: **09.05.2007**
(21) Anmeldenummer: 02787321.5
(22) Anmeldetag: 02.12.2002
(51) Int. Cl.: A61B 17/80

(54) **IMPLANTAT FÜR DIE KNOCHENFIXATION**
IMPLANT FOR FIXING BONES
IMPLANT POUR FIXATION OSSEUSE

(43) Veröffentlichungstag der Anmeldung: 31.08.2005
(73) Patentinhaber: Synthes GmbH, 4436 Oberdorf (CH)
(72) Erfinder: MATHIEU, Claude, CH-8002 Zurich (CH); FRIGG, Robert, CH-2544 Bettlach (CH); SPICHIGER, Marco, CH-2544 Bettlach (CH); LECHMANN, Beat, CH-2544 Bettlach (CH)
(74) Vertreter: Lusuardi, Werther
(86) Internationale Anmeldenummer: PCT/CH2002/000650
(87) Internationale Veröffentlichungsnummer: WO 2004/049962

(56) Entgegenhaltungen:
- WO-A-03/055401
- DE-A- 1 949 923
- FR-A- 2 726 461
- GB-A- 1 300 449
- US-A- 5 108 399
- US-A- 5 578 034
- US-A- 6 004 323
- US-A1- 2001 021 851
- US-A1- 2002 082 603
- US-B1- 6 206 881

## Beschreibung

Die Erfindung bezieht sich auf ein Implantat für die Knochenfixation, insbesondere eine Knochenplatte gemäss dem Oberbegriff des Patentanspruchs 1.

Knochenplatten aus Metallen oder Metallegierungen einerseits und aus Kunststoffen, insbesondere resorbierbaren Polymeren anderseits sind bereits bekannt. Die metallischen Knochenplatten sind aber zu steif und die weniger steifen KunststoffPlatten, insbesondere solche aus resorbierbaren Kunststoffen, weisen eine zu niedrige Festigkeit auf. Zudem ergeben sich Probleme beim Verbinden von Kunststoffplatten mit metallischen Knochenschrauben, insbesondere sind die Verbindungen teilweise nicht stabil genug.

Dokument US-A-5,057,111 offenbart ein Implantat nach dem Oberbegriff des Anspruchs 1.

Hier will die Erfindung Abhilfe schaffen. Der Erfindung liegt die Aufgabe zugrunde, ein Implantat zu schaffen, welches einerseits weniger steif ist und anderseits keine Probleme mit der Schrauben-Implantat-Interface bietet.

Die Erfindung löst die gestellte Aufgabe mit einem Implantat, welches die Merkmale des Anspruchs 1 aufweist.

Bei einer Ausführungsform des Implantates ist die Umrandung ring- oder hülsenförmig geformt. Die Aussenform der Umrandung kann aber auch polygonal, z.B. rechteckig ausgeführt sein.

Vorzugsweise ist das Implantat als Knochenplatte ausgebildet, welche eine für den Knochenkontakt geeignete Unterseite und eine Oberseite aufweist, wobei die Durchgänge die Oberseite mit der Unterseite verbinden.

Die Umrandung besteht vorzugsweise aus einem Metall oder einer Metallegierung und das die Umrandung umgebende Material ist vorzugsweise ein Kunststoff. Dadurch muss die Platte weniger Last tragen. Zudem ist die Platte auch weniger steif, was zu einer besseren Heilung des Knochens führt gemäss dem Wolffschen Gesetz und schliesslich ist sie auch weniger röntgenopak, so dass weniger Artefakte in Röntgenbildern, CT oder MRI auftreten und eine bessere Verfolgung des Heilungsprozesses möglich ist.

Die Umrandung kann aus einem Metall oder einer Metallegierung bestehen und das die Umrandung umgebende Material kann ein Kunststoff sein.

Als Kunststoff eignet sich insbesondere PEEK oder verwandte Polymere aus der Familie der Polyaryletherketone (PAEK). Der Kunststoff kann zudem verstärkt werden, z.B. mit Kohlefasern oder PEEK-Fasern.

Geeignete Metalle sind z.B. Titan, Titanlegierungen oder Implantatstahl.

Die aus Kunststoff bestehenden Bauteile des Implantates sind vorteilhafterweise mit einer Titanschicht oder einer Hydroxylapatit-Schicht überzogen. Der Vorteil der Beschichtung besteht darin, dass dadurch kein direkter Kontakt zwischen Metall und Knochen möglich ist und dass die gleiche Oberfläche wie bei einer Metallplatte erreicht wird, aber mit den mechanischen Eigenschaften eines Kunststoffs.

Bei einer besonderen Ausführungsform weist die Umrandung eine als Zielhilfe für ein Knochenfixationsmittel geeignete hülsenförmige Verlängerung auf. Die Verlängerung kann an der Umrandung angeformt sein und beide - Umrandung und hülsenförmige Verlängerung - bestehen vorzugsweise aus Kunststoff.

Eine besonders einfache Herstellung des Implantates ergibt sich, wenn die Umrandung aus Metall oder eine Metallegierung besteht und im die Umrandung umgebenden Kunststoff, gegenüber der Oberseite, vertieft eingelassen ist. Alternativ dazu kann die Umrandung im umgebenden Kunststoff, gegenüber der Oberseite, erhöht eingelassen sein.

Die Ebene, welche die Umrandung enthält oder an dieser anliegt kann bei einer Ausführungsform einen Winkel im Bereich von 0,1° bis 20,0° zur Plattenebene einschliessen, so dass dann auch die Durchgänge (bzw. die Achsen der Durchgänge) gegenüber der Vertikalen auf der Plattenebene einen solchen Winkel einschliessen.

Bei einer Ausführungsform weist das Implantat - insbesondere wenn es sich um eine Knochenplatte handelt - mindestens zwei das Implantat durchstossende Durchgänge auf, welche zur Aufnahme eines Knochenfixationsmittels, insbesondere einer Knochenschraube geeignet sind.

Bei einer weiteren Ausführungsform sind mindestens zwei der das Implantat durchstossenden Durchgänge mit einer periphere Umrandung versehen, die aus einem anderen Material bestehen als das die Umrandung umgebende Material des Implantats. Vorzugsweise sind dann die peripheren Umrandungen von mehreren das Implantat durchstossenden Durchgängen untereinander einstückig verbunden, was eine einfachere Herstellung des Implantats und eine bessere Verankerung der Umrandungen im umgebenden Kunststoffmaterial ermöglicht.

Bei mehreren peripheren Umrandungen können diese auch in Form eines Gitters untereinander verbunden sein.

Das Implantat kann mit geeigneten, in die Durchgänge einführbaren Knochenfixationsmittel, vorzugsweise in Form von Knochenschrauben am zu behandelnden Knochen befestigt werden. Dabei können die Knochenschrauben monoaxial oder polyaxial gegenüber dem Implantat eingebracht werden. Die Verbindung der Knochenschrauben mit dem Implantat kann entweder winkelstabil oder nicht- winkelstabil erfolgen.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Aufsicht auf eine Knochenplatte aus Kunststoff mit vier Plattenbohrungen, welche eine metallische, rechteckige Umrandung aufweisen;
Fig. 2 einen Längsschnitt durch die Knochenplatte nach Fig. 1 längs der Linie II-II;
Fig. 3 eine perspektivische Ansicht einer modifizierten Knochenplatte mit hülsenförmigen Umrandungen;
Fig. 4 eine Aufsicht auf die Knochenplatte nach Fig. 3; und
Fig. 5 eine perspektivische Ansicht einer modifizierten Knochenplatte mit einer hülsenförmigen Verlängerung an einem der Plattenlöcher.

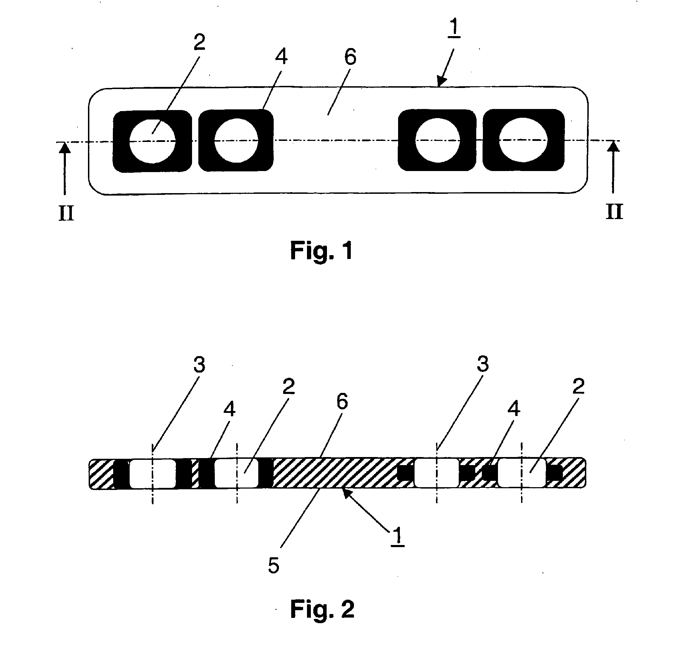

Das in den Fig. 1 und 2 dargestellte Implantat 1 ist eine Knochenplatte, welche zur Hauptsache aus biokompatiblem Kunststoff, z.B. Polyetheretherketon (PEEK) besteht und vier Durchgänge 2 in Form von Kreisbohrungen mit der Achse 3 zur Aufnahme von (zeichnerisch nicht dargestellten) Knochenschrauben aufweist. Die Durchgänge 2 verbinden die zur Anlage an den Knochen geeignete Unterseite 5 der Knochenplatte mit ihrer Oberseite 6.
Die Durchgänge 2 sind dabei in Form von rechteckförmigen Plättchen aus Metall eingelassen, welche als Umrandungen 4 der Durchgänge 2 aufgefasst werden können. Die beispielsweise aus Titan bestehenden Umrandungen 4 sind herstellungsmässig im Spritzguss in das umgebende Kunststoff-Material 7 (z.B. PEEK) eingearbeitet und formschlüssig fest mit dem Kunststoffmaterial 7 verbunden.
Auf der rechten Seite der Fig. 2 ist eine Variante der Einlassung der Umrandungen 4 in das Kunststoffmaterial 7 dargestellt, bei welcher die metallischen Plättchen weniger hoch ausgebildet sind als auf der linken Seite der Fig. 2 , so dass sie gegenüber der Oberseite 6 und der Unterseite 5 vertieft eingelassen sind und deshalb vom Kunststoffmaterial 7 umschlossen werden.

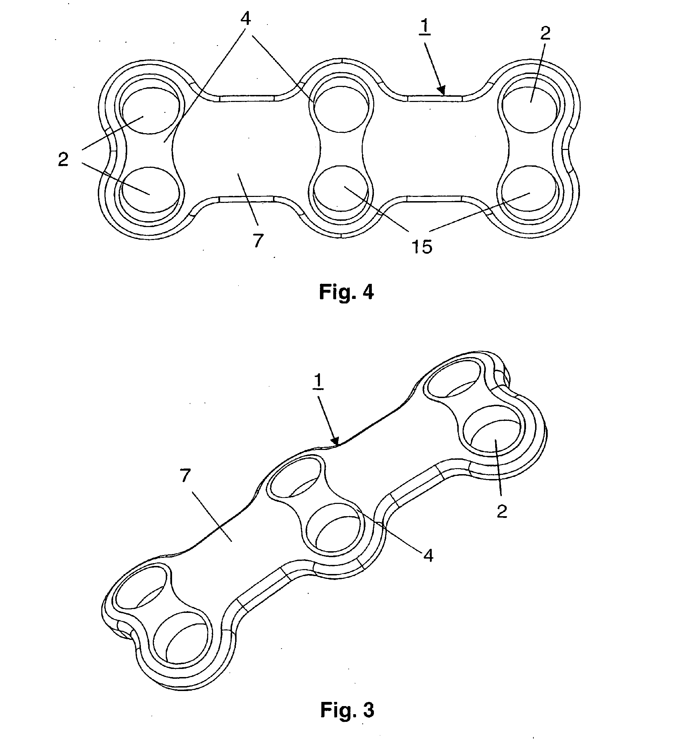

Bei einer anderen, in den Fig. 3 und 4 dargestellten Ausführungsform ist das Implantat 1 ebenfalls eine Knochenplatte. Sie besteht aus PEEK, welches mit PEEK-Fasern verstärkt ist. Im Unterschied zur Ausführungsform gemäss den Fig. 1 und 2, sind hier jeweils zwei Umrandungen 4 aus Titan einstückig miteinander verbunden und als Ganzes im umgebenden PEEK-Material 7 spritzgussmässig eingelassen. Die Aussenform der einzelnen Umrandungen 4 ist dabei kreis-, bzw. hülsenförmig .

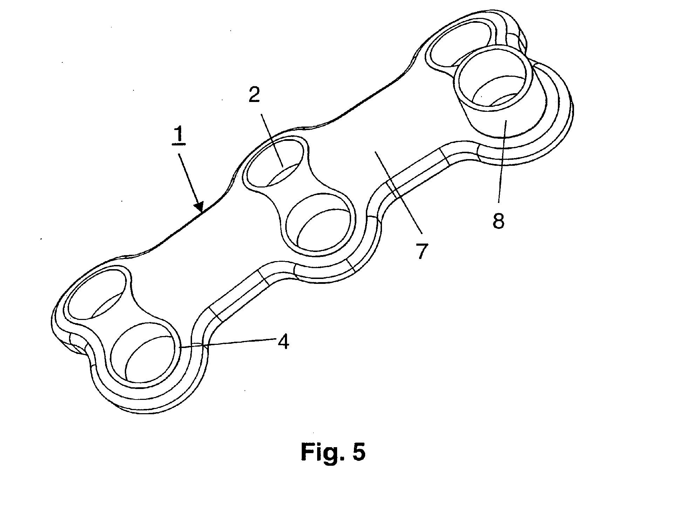

Bei der in Fig. 5 dargestellten Modifikation ist an einer der Umrandungen 4 eine als Zielhilfe für ein Knochenfixationsmittel, z.B. eine Knochenschraube geeignete hülsenförmige Verlängerung 8 angeformt.

## Patentansprüche

1. Implantat (1) für die Knochenfixation, welches
A) aus einer Kombination der beiden Materialien Metall und Kunststoff besteht; und
B) mindestens einen das Implantat (1) durchstossenden Durchgang (2) mit einer Achse (3) zur Aufnahme eines Knochenfixationsmittels aufweist; und
C) der Durchgang (2) mit einer peripheren Umrandung (4) versehen ist, die aus einem anderen Material besteht als das die Umrandung (4) umgebende Material des Implantats (1); **dadurch gekennzeichnet, dass**
D) die periphere Umrandung (4) formschlüssig fest mit dem Kunststoffmaterial (7) des Implantats verbunden ist.

2. Implantat (1) nach Anspruch 1, **dadurch gekennzeichnet, dass** die Umrandung (4) ring- oder hülsenförmig geformt ist.

3. Implantat (1) nach Anspruch 1, **dadurch gekennzeichnet, dass** die Umrandung (4) eine polygonale Aussenform aufweist.

4. Implantat (1) nach einem der Ansprüche 1 bis 3, **dadurch gekennzeichnet, dass** es als Knochenplatte ausgebildet ist, welche eine für den Knochenkontakt geeignete Unterseite (5) und eine Oberseite (6) aufweist, wobei der Durchgang (2) die Oberseite (6) mit der Unterseite (5) verbindet.

5. Implantat (1) nach einem der Ansprüche 1 - 4, **dadurch gekennzeichnet, dass** die Umrandung (4) aus einem Metall oder eine Metallegierung besteht und das die Umrandung (4) umgebende Material ein Kunststoff ist.

6. Implantat (1) nach einem der Ansprüche 1 - 5, **dadurch gekennzeichnet, dass** der Kunststoff aus der Gruppe der Polyaryletherketone (PAEK) ausgewählt ist.

7. Implantat (1) nach einem der Ansprüche 1 - 5, **dadurch gekennzeichnet, dass** der Kunststoff PEEK ist.

8. Implantat (1) nach einem der Ansprüche 1 - 7, **dadurch gekennzeichnet, dass** der Kunststoff verstärkt ist, vorzugsweise mit Kohlefasern oder PEEK-Fasern.

9. Implantat (1) nach einem der Ansprüche 1 - 8, **dadurch gekennzeichnet, dass** das Metall Titan, eine Titanlegierung oder Implantatstahl ist.

10. Implantat (1) nach einem der Ansprüche 1 - 9, **dadurch gekennzeichnet, dass** die aus Kunststoff bestehenden Bauteile des Implantates mit einer Titanschicht oder einer Hydroxylapatit-Schicht überzogen sind.

11. Implantat (1) nach einem der Ansprüche 1 - 10, **dadurch gekennzeichnet, dass** die Umrandung (4) eine als Zielhilfe für ein Knochenfixationsmittel geeignete hülsenförmige Verlängerung (8) aufweist.

12. Implantat (1) nach Anspruch 11, **dadurch gekennzeichnet, dass** die Verlängerung (8) an der Umrandung (4) angeformt ist und beide aus Kunststoff bestehen.

13. Implantat (1) nach einem der Ansprüche 1 - 11, **dadurch gekennzeichnet, dass** die Umrandung (4) aus Metall oder eine Metallegierung besteht und im die Umrandung (4) umgebenden Kunststoff, gegenüber der Oberseite (6), vertieft eingelassen ist.

14. Implantat (1) nach einem der Ansprüche 1 - 11, **dadurch gekennzeichnet, dass** die Umrandung (4) aus Metall oder eine Metallegierung besteht und im die Umrandung (4) umgebenden Kunststoff, gegenüber der Oberseite (6), erhöht eingelassen ist.

15. Implantat (1) nach einem der Ansprüche 1 - 14, **dadurch gekennzeichnet, dass** die Ebene, welche die Umrandung (4) enthält oder an dieser anliegt einen Winkel im Bereich von 0,1° bis 20,0° zur Plattenebene einschliesst.

16. Implantat (1) nach einem der Ansprüche 1 - 14, **dadurch gekennzeichnet, dass** es mindestens zwei das Implantat (1) durchstossende Durchgänge (2) mit einer Achse (3) zur Aufnahme eines Knochenfixationsmittels aufweist.

17. Implantat (1) nach einem der Ansprüche 1 - 16, **dadurch gekennzeichnet, dass** mindestens zwei der das Implantat (1) durchstossenden Durchgänge (2) mit einer periphere Umrandung (4) versehen sind, die aus einem anderen Material bestehen als das die Umrandung (4) umgebende Material des Implantats (1).

18. Implantat nach einem der Ansprüche 1 - 17, **dadurch gekennzeichnet, dass** die peripheren Umrandungen (4) von mehreren das Implantat (1) durchstossenden Durchgängen (2) untereinander einstückig verbunden sind.

19. Implantat nach einem der Ansprüche 1 - 18, **dadurch gekennzeichnet, dass** mehrere periphere Umrandungen (4) in Form eines Gitters untereinander verbunden sind.

20. Implantat nach einem der Ansprüche 1 - 19, **dadurch gekennzeichnet, dass** es mindestens ein in die Durchgänge (2) einführbares Knochenfixationsmittel, vorzugsweise eine Knochenschraube umfasst, welches gegenüber dem Implantat polyaxial positionierbar ist.

21. Implantat nach einem der Ansprüche 1 - 20, **dadurch gekennzeichnet, dass** es mindestens ein in die Durchgänge (2) einführbares Knochenfixationsmittel, vorzugsweise eine Knochenschraube umfasst, welches winkelstabil mit dem Implantat verbindbar ist.

## Claims

1. Implant (1) for the bone fixation
A) consisting of a combination of the two materials metal and plastic; and
B) at least one passage (2) running through the implant (1) with an axis (3) for receiving a bone fixation device; and
C) the passage (2) is provided with a peripheral rim (4) that is made of a different material that the implant material (1) surrounding the rim (4);
**characterised in that**
D) the peripheral rim (4) is linked form-fittingly rigidly to the plastic material (7) of the implant.

2. Implant (1) in accordance with claim 1, **characterised in that** the rim (4) is ringshaped or sleeve-shaped.

3. Implant (1) in accordance with claim 1, **characterised in that** the rim (4) is provided with a polygonal external form.

4. Implant (1) in accordance with one of the claims 1 to 3, **characterised in that** it is configured as a bone plate that is provided with a bottom side (5) suitable for bone contact and an upper side (6), wherein the passage (2) connects the upper side (6) to the bottom side (5).

5. Implant (1) in accordance with one of the claims 1 - 4, **characterised in that** the rim (4) is made of a metal or a metal alloy and that the material surrounding the rim (4) is plastic.

6. Implant (1) in accordance with one of the claims 1 - 5, **characterised in that** the plastic is chosen from the Polyaryletherketone (PAEK) family.

7. Implant (1) in accordance with one of the claims 1 - 5, **characterised in that** PEEK is used as plastic.

8. Implant (1) in accordance with one of the claims 1 - 7, **characterised in that** the plastic is reinforced, preferably with carbon fibres or PEEK fibres.

9. Implant (1) in accordance with one of the claims 1 - 8, **characterised in that** the metal titanium is a titanium alloy or implant steel.

10. Implant (1) in accordance with one of the claims 1 - 9, **characterised in that** the elements of the implant that are made of plastic are covered with a coating of titanium or a Hydroxylapatite layer.

11. Implant (1) in accordance with one of the claims 1 - 10, **characterised in that** the rim (4) is provided with a sleeve-shaped extension (8) as target aid for a bone fixation device.

12. Implant (1) in accordance with claim 11, **characterised in that** the extension (8) is formed on the rim (4) and both are made of plastic.

13. Implant (1) in accordance with one of the claims 1-11, **characterised in that** the rim (4) is made of a metal or a metal alloy and is set lowered in the plastic surrounding the rim (4), relative to the upper side (6).

14. Implant (1) in accordance with one of the claims 1 - 11, **characterised in that** the rim (4) is made of a metal or a metal alloy and set raised in the plastic surrounding the rim (4), relative to the upper side (6).

15. Implant (1) in accordance with one of the claims 1 - 14, **characterised in that** the level containing or laid on the rim (4) has an angle in the range 0,1° to 20,0° to the plate level.

16. Implant (1) in accordance with one of the claims 1 - 15, **characterised in that** it is provided with at least two passages (2) running through the implant (1) with an axis (3) for receiving a bone fixation device.

17. Implant (1) in accordance with one of the claims 1 - 16, **characterised in that** at least two of the passages (2) running through the implant (1) are provided with a peripheral rim (4) that is made of a different material than the material of the implant (1) surrounding the rim (4).

18. Implant in accordance with one of the claims 1 - 17, **characterised in that** the peripheral rims (4) of several passages (2) running through the implant (1) are joined to each other in one piece.

19. Implant in accordance with one of the claims 1 - 18, **characterised in that** a plurality of peripheral rims (4) are joined to each other in the form of a grid.

20. Implant in accordance with one of the claims 1 - 19, **characterised in that** it comprises at least one bone fixation device that can be inserted into the passages (2), preferably a bone screw, which can be positioned in poly-axially in relation to the implant.

21. Implant in accordance with one of the claims 1 - 20, **characterised in that** it comprises at least one bone fixation device that can be inserted into the passages (2), preferably a bone screw, which can be connected with the implant with stable angle.

## Revendications

1. Implant (1) pour fixation osseuse, qui
A) est composé d'une combinaison des deux matériaux métal et matière plastique ; et qui
B) présente au moins un passage (2) traversant l'implant (1) avec un axe (3) pour loger un moyen de fixation osseuse ; et
C) le passage (2) est pourvu d'un bord périphérique (4) fait d'un matériau différent du matériau de l'implant (1) entourant le bord (4) ;
**caractérisé en ce que**
D) le bord périphérique (4) est fermement relié par emboîtement à la matière plastique (7) de l'implant.

2. Implant (1) selon la revendication 1, **caractérisé en ce que** le bord (4) est réalisé sous forme d'anneau ou de douille.

3. Implant (1) selon la revendication 1, **caractérisé en ce que** le bord (4) présente une forme externe polygonale.

4. Implant (1) selon l'une quelconque des revendications 1 à 3, **caractérisé en ce qu'**il est réalisé sous forme de plaque d'ostéosynthèse, laquelle présente une face inférieure (5) appropriée au contact avec l'os et une face supérieure (6), dans lequel le passage (2) relie la face supérieure (6) à la face inférieure (5).

5. Implant (1) selon l'une quelconque des revendications 1 à 4, **caractérisé en ce que** le bord (4) est fait d'un métal ou d'un alliage métallique et **en ce que** le matériau entourant le bord (4) est fait de matière plastique.

6. Implant (1) selon l'une quelconque des revendications 1 à 5, **caractérisé en ce que** la matière plastique est choisie dans le groupe des polyaryléthercétones (PAEK).

7. Implant (1) selon l'une quelconque des revendications 1 à 5, **caractérisé en ce que** la matière plastique est la polyéther-éther-cétone (PEEK).

8. Implant (1) selon l'une quelconque des revendications 1 à 7, **caractérisé en ce que** la matière plastique est renforcée, de préférence avec des fibres de carbone ou des fibres de PEEK.

9. Implant (1) selon l'une quelconque des revendications 1 à 8, **caractérisé en ce que** le métal est le titane, un alliage du titane ou un acier pour implants.

10. Implant (1) selon l'une quelconque des revendications 1 à 9, **caractérisé en ce que** les éléments de l'implant faits de matière plastique sont revêtus d'une couche de titane ou d'hydroxyapatite.

11. Implant (1) selon l'une quelconque des revendications 1 à 10, **caractérisé en ce que** le bord (4) présente un prolongateur (8) en forme de douille approprié en tant qu'auxiliaire de visée pour un moyen de fixation osseuse.

12. Implant (1) selon la revendication 11, **caractérisé en ce que** le prolongateur (8) est moulé sur le bord (4) et **en ce que** les deux sont faits de matière plastique.

13. Implant (1) selon l'une quelconque des revendications 1 à 11, **caractérisé en ce que** le bord (4) est fait de métal ou d'un alliage métallique et **en ce qu'**il est encastré dans la matière plastique entourant le bord (4) en retrait par rapport à la face supérieure (6).

14. Implant (1) selon l'une quelconque des revendications 1 à 11, **caractérisé en ce que** le bord (4) est fait de métal ou d'un alliage métallique et **en ce qu'**il est encastré dans la matière plastique entourant le bord (4) en saillie par rapport à la face supérieure (6).

15. Implant (1) selon l'une quelconque des revendications 1 à 14, **caractérisé en ce que** le plan qui contient le bord (4) ou qui est adjacent à celui-ci renferme un angle de 0,1° à 20,0° par rapport au plan de la plaque.

16. Implant (1) selon l'une quelconque des revendications 1 à 14, **caractérisé en ce qu'**il présente au moins deux passages (2) traversant l'implant (1) avec un axe (3) pour loger un moyen de fixation osseuse.

17. Implant (1) selon l'une quelconque des revendications 1 à 16, **caractérisé en ce qu'**au moins deux des passages (2) traversant l'implant (1) sont pourvus d'un bord périphérique (4) qui est fait d'un matériau différent du matériau de l'implant (1) entourant le bord (4).

18. Implant selon l'une quelconque des revendications 1 à 17, **caractérisé en ce que** les bords périphériques (4) de plusieurs passages (2) traversant l'implant (1) sont reliés entre eux en formant une seule pièce.

19. Implant selon l'une quelconque des revendications 1 à 18, **caractérisé en ce que** plusieurs bords périphériques (4) sont reliés entre eux sous la forme d'une grille.

20. Implant selon l'une quelconque des revendications 1 à 19, **caractérisé en ce qu'**il comprend au moins un moyen de fixation osseuse pouvant être inséré dans les passages (2), de préférence une vis à os, lequel peut être positionné de manière polyaxiale par rapport à l'implant.

21. Implant selon l'une quelconque des revendications 1 à 20, **caractérisé en ce qu'**il comprend au moins un moyen de fixation osseuse pouvant être inséré dans les passages (2), de préférence une vis à os, lequel peut être relié à l'implant à angle fixe.
